(19) Europäisches Patentamt / European Patent Office / Office européen des brevets

(11) **EP 3 730 036 A1**

(12) **EUROPÄISCHE PATENTANMELDUNG**

(43) Veröffentlichungstag:
**28.10.2020 Patentblatt 2020/44**

(51) Int Cl.:
*A61B 3/103* (2006.01)    *A61B 3/032* (2006.01)

(21) Anmeldenummer: **19170558.1**

(22) Anmeldetag: **23.04.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Carl Zeiss Vision International GmbH 73430 Aalen (DE)**

(72) Erfinder:
- **Ohlendorf, Arne**
  **72072 Tübingen (DE)**
- **Leube, Alexander**
  **72072 Tübingen (DE)**
- **Wahl, Siegfried**
  **73072 Donzdorf (DE)**

(74) Vertreter: **Altmann Stößel Dick Patentanwälte PartG mbB**
**Dudenstrasse 46**
**68167 Mannheim (DE)**

(54) **BESTIMMUNG EINES REFRAKTIONSFEHLERS EINES AUGES**

(57) Die vorliegende Erfindung betrifft ein Verfahren (210), eine Vorrichtung (110) und ein Computerprogramm zur Bestimmung eines Refraktionsfehlers eines Auges (112) eines Nutzers (114), sowie ein Verfahren zur Herstellung eines Brillenglases für das Auge (112) des Nutzers (114). Das Verfahren (210) zur Bestimmung des Refraktionsfehlers des Auges (112) des Nutzers (114) umfasst die folgenden Schritte:

a) Darstellen eines Zeichens (122) auf einem Bildschirm (120), wobei ein Parameter des auf dem Bildschirm (120) dargestellten Zeichens (122) verändert wird;

b) Erfassen einer Reaktion des Nutzers (114) in Abhängigkeit von dem auf dem Bildschirm (120) dargestellten Zeichen (122);

c) Feststellen eines Zeitpunkts, zu dem sich aus der Reaktion des Nutzers (114) eine Erkennbarkeit des auf dem Bildschirm (120) dargestellten Zeichens (122) für den Nutzer (114) ergibt; und

d) Bestimmen eines Wertes (220) für den Refraktionsfehler des Auges (112) des Nutzers (114) aus dem zu dem Zeitpunkt festgelegten Parameter,

wobei das auf dem Bildschirm (120) dargestellte Zeichen (122) ein periodisches Muster (124) ist, wobei der Parameter des auf dem Bildschirm (120) dargestellten Musters (124) mindestens eine Ortsfrequenz umfasst, und dass der Wert (220) für den Refraktionsfehler aus der zu dem Zeitpunkt festgelegten Ortsfrequenz des Musters (124) bestimmt wird.

Fig. 1

EP 3 730 036 A1

**Beschreibung**

Gebiet der Erfindung

Die vorliegende Erfindung betrifft ein Verfahren, eine Vorrichtung und ein Computerprogramm zur Bestimmung eines Refraktionsfehlers eines Auges eines Nutzers, sowie ein Verfahren zur Herstellung eines Brillenglases für das Auge des Nutzers.

Stand der Technik

Aus dem Stand der Technik sind Verfahren zur Bestimmung von Refraktionsfehlern eines Auges eines Nutzers bekannt. Der Begriff "Refraktion" bezeichnet hierbei eine Lichtbrechung im Auge des Nutzers, die ein durch die Pupille in das Innere des Auges einfallender Lichtstrahl erfährt. Zur Bestimmung der Refraktion werden üblicherweise Sehzeichen, bevorzugt in Form von Zahlen, Buchstaben oder Symbolen, auf einer Tafel oder einem Bildschirm in einer festgelegten Größe für eine gegebene Entfernung bereitgestellt, die der Nutzer betrachtet. Durch ein Vorhalten einer Anzahl von Brillengläsern mit bekannten Eigenschaften sowie durch eine Führung des Nutzers in einem Frageprozess kann subjektiv bestimmt werden, welche Defokussierung das Auge des Nutzers aufweist und welche sphärozylindrische Ausgestaltung des Brillenglases zu einem weitgehenden Ausgleich der Refraktionsfehler des Auges und damit zu einer möglichst optimalen Bildqualität für den Nutzer führt. Die Defokussierung des Auges des Nutzers kann zu einer Fehlsichtigkeit (Ametropie) des Nutzers führen, insbesondere zu einer Kurzsichtigkeit (Myopie) oder zu einer Weitsichtigkeit (Hyperopie) des Nutzers.

US 2014/268060 A1 offenbart eine Vorrichtung und ein Verfahren zur Bestimmung der Refraktion eines Auges sowie eines Astigmatismus unter Verwendung eines Computer-Bildschirms. Hierzu wird ein Sehzeichen auf dem Bildschirm dargestellt und durch Veränderung der auf dem Bildschirm dargestellten Größe des Sehzeichens ein Wert für die Größe des Sehzeichens ermittelt, bei welchem das Sehzeichen für den Nutzer gerade nicht mehr erkennbar ist.

WO 2018/077690 A1 offenbart Vorrichtungen und ein Computerprogramm, mit welchem die sphärozylindrische Refraktion eines Auges bestimmt werden kann. Hierzu wird eine Komponente mit einer einstellbaren Optik bereitgestellt, welche über eine BrechkraftEinstelleinrichtung hinsichtlich ihrer Brechkraft verstellt werden kann. Aus der Einstellung der Brechkrafteinstelleinrichtung bei verschiedenen Orientierungen einer Vorzugsrichtung der Optik oder einer Vorzugsrichtung von Sehzeichen wird dann die sphärozylindrische Refraktion bestimmt.

Aufgabe der Erfindung

Insbesondere ausgehend von der Offenbarung der US 2014/268060 A1 besteht die Aufgabe der vorliegenden Erfindung darin, ein Verfahren, eine Vorrichtung und ein Computerprogramm zur Bestimmung eines Refraktionsfehlers eines Auges eines Nutzers sowie ein Verfahren zur Herstellung eines Brillenglases für das Auge des Nutzers bereitzustellen, welche die aufgeführten Nachteile und Einschränkungen des Standes der Technik zumindest teilweise überwinden.

Insbesondere sollen es das vorliegende Verfahren, Vorrichtung und Computerprogramm ermöglichen, eine Defokussierung des Auges des Nutzers zu ermitteln, um hieraus den Refraktionsfehler des Auges des Nutzers zu bestimmen. Die Ermittlung der Defokussierung des Auges des Nutzers soll hierbei ohne Spezialgeräte erfolgen können und daher auch von Laien ausgeführt werden.

Offenbarung der Erfindung

Diese Aufgabe wird gelöst durch ein Verfahren, ein Computerprogramm und eine Vorrichtung zur Bestimmung eines Refraktionsfehlers eines Auges eines Nutzers sowie ein Verfahren zur Herstellung eines Brillenglases für das Auge des Nutzers mit den Merkmalen der unabhängigen Patentansprüche. Bevorzugte Ausgestaltungen, welche einzeln oder in Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben dem durch diese Begriffe eingeführten Merkmal, keine weiteren Merkmale vorhanden sind oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist, d.h. auf eine Situation, in welcher A ausschließlich aus B besteht, als auch auf die Situation, in welcher, zusätzlich zu B, ein oder mehrere weitere Elemente in A vorhanden sind, beispielsweise Element C, Elemente C und D oder sogar weitere Elemente.

In einem ersten Aspekt betrifft die vorliegende Erfindung ein Verfahren zur Bestimmung eines Refraktionsfehlers eines Auges eines Nutzers. Das Verfahren umfasst die folgenden Schritte a) bis d), vorzugsweise in der angegebenen Reihenfolge. Auch eine andere Reihenfolge ist grundsätzlich möglich. Insbesondere ist auch eine ganz oder teilweise zeitgleiche Ausführung der Verfahrensschritte möglich. Weiterhin können einzelne, mehrere oder alle Schritte des Verfahrens wiederholt, insbesondere mehr als einmal, ausgeführt werden. Das Verfahren kann, zusätzlich zu den genannten Verfahrensschritten auch weitere Verfahrensschritte umfassen.

Das Verfahren zur Bestimmung eines Refraktionsfehlers eines Auges eines Nutzers umfasst die Schritte:

a) Darstellen eines Zeichens auf einem Bildschirm, wobei ein Parameter des auf dem Bildschirm dargestellten Zeichens verändert wird;
b) Erfassen einer Reaktion des Nutzers in Abhängigkeit von dem auf dem Bildschirm dargestellten Zeichen;
c) Feststellen eines Zeitpunkts, zu dem sich aus der Reaktion des Nutzers eine Erkennbarkeit des auf dem Bildschirm dargestellten Zeichens für den Nutzer ergibt; und
d) Bestimmen eines Wertes für den Refraktionsfehler des Auges des Nutzers aus dem zu dem Zeitpunkt festgelegten Parameter,

wobei das auf dem Bildschirm dargestellte Zeichen ein periodisches Muster ist, wobei der Parameter des auf dem Bildschirm dargestellten Musters mindestens eine Ortsfrequenz umfasst, und wobei der Wert für den Refraktionsfehler aus der zu dem Zeitpunkt festgelegten Ortsfrequenz des Musters bestimmt wird.

Das hier vorgeschlagene Verfahren zur Bestimmung eines Refraktionsfehlers eines Auges eines Nutzers eignet sich insbesondere zur Verwendung in einem Verfahren zur Herstellung eines Brillenglases für das Auge des betreffenden Nutzers. Unter einem "Brillenglas" wird gemäß Norm DIN EN ISO 13666:2013-10, im Folgenden auch als die "Norm" bezeichnet, Abschnitte 8.1.1 und 8.1.2 eine optische Linse verstanden, welche im Rahmen der vorliegenden Erfindung zur Korrektur von Fehlsichtigkeiten des Auges dienen soll, wobei die optische Linse vor dem Auge des Nutzers, aber nicht in Kontakt mit dem Auge getragen wird.

Der Begriff der "Brille" bezeichnet im Rahmen der vorliegenden Erfindung ein beliebiges Element, welches zwei einzelne Brillengläser und eine Brillenfassung umfasst, wobei das Brillenglas zur Einbringung in eine Brillenfassung vorgesehen ist, welche von einem Nutzer der Brille ausgewählt wird. Anstelle des hier verwendeten Begriffs des "Nutzers" kann gleichbedeutend auch einer der Begriffe "Subjekt", "Brillenträger", "Benutzer" oder "Proband" verwendet werden.

Insbesondere kann aus einer Bestimmung der bei dem Nutzer auftretenden Refraktionsfehler eine sphärozylindrische Linse ermittelt werden, die als Brillenglas dazu benutzt wird, die als Defokussierung des Auges auftretenden Refraktionsfehlern derart zu kompensieren, dass eine möglichst optimale Bildqualität für den Nutzer erzielen werden kann. Zur Beschreibung der sphärozylindrischen Linse eignen sich verschiedene Darstellungsweisen. Die Norm legt in Abschnitt 11.2 einen so genannten "sphärischen Brechwert" fest, welcher als Wert für einen Scheitelbrechwert eines Brillenglases mit sphärischer Wirkung oder für den jeweiligen Scheitelbrechwert in einem von zwei Hauptschnitten des Brillenglases mit astigmatischer Wirkung definiert ist. Nach der Norm, 9.7.1 und 9.7.2 ist der "Scheitelbrechwert" als Kehrwert einer paraxialen Schnittweite eines bildseitigen Brennpunktes, jeweils gemessen in Metern, festgelegt. Das sphärozylindrische Brillenglas mit astigmatischer Wirkung vereinigt gemäß der Norm, Abschnitt 12, ein paraxiales, paralleles Lichtbündel in zwei getrennten, zueinander senkrecht stehenden Brennlinien, und besitzt daher nur in den beiden Hauptschnitten einen Scheitelbrechwert. Die "astigmatische Wirkung" ist hierbei durch Zylinderstärke und Achslage festgelegt. Hierbei stellt die "Zylinderstärke" gemäß der Norm, 12.5 den Betrag einer "astigmatischen Differenz" dar, welche die Differenz zwischen den Scheitelbrechwerten in den beiden Hauptschnitten angibt. Die "Achslage" bezeichnet gemäß der Norm, 12.6 eine Richtung des Hauptschnittes, dessen Scheitelbrechwert als Referenzwert herangezogen wird. Schließlich wird nach der Norm, 12.8 die "Stärke" des Brillenglases mit astigmatischer Wirkung mittels drei Werten, umfassend die Scheitelbrechwerte jedes der beiden Hauptschnitte und die Zylinderstärke, angegeben.

Nach L. N. Thibos, W. Wheeler und D. Horner (1997), Power Vectors: An Application of Fourier Analysis to the Description and Statistical Analysis of Refractive Error, Optometry and Vision Science 74 (6), S. 367-375, eignet sich zur Beschreibung einer beliebigen sphärozylindrischen Linse die Angabe eines Sehstärkevektors, welcher durch genau einen Punkt in einem dreidimensionalen dioptrischen Raum beschrieben werden kann, wobei der dreidimensionale dioptrische Raum durch Koordinaten aufgespannt werden kann, welche der Sehstärke und der Zylinderstärke entsprechen bzw. damit korreliert sind.

Gemäß Schritt a) des vorliegenden Verfahrens erfolgt eine Darstellung eines Zeichens auf einem Bildschirm, wobei ein Parameter des auf dem Bildschirm dargestellten Zeichens verändert wird. Der Begriff "Bildschirm" bezeichnet hierbei eine elektronisch ansteuerbare Anzeige, welche über eine zwei-dimensionale Ausdehnung verfügt, wobei das gewünschte Zeichen mit weitgehend frei wählbaren Parametern an einer beliebigen Stelle innerhalb der Ausdehnung darstellbar ist. Der Bildschirm kann hierbei bevorzugt ausgewählt sein aus einen Monitor, einem Screen oder einem Display. Vorzugsweise kann der Bildschirm hierbei von einen mobilen Kommunikationsgerät umfasst sein. Der Begriff des "mobilen Kommunikationsgeräts" umfasst hierbei insbesondere ein Mobiltelefon (Handy), ein Smartphone oder ein Tablet. Andere Arten von mobilen Kommunikationsgeräten sind jedoch denkbar. Auf diese Weise kann das vorliegende Verfahren zur Bestimmung eines Refraktionsfehlers des Auges an einem beliebigen Ort durchgeführt werden. Andere Arten von Bildschirmen sind jedoch ebenfalls möglich.

Der Begriff des "Zeichens" betrifft einerseits Sehzeichen, insbesondere Buchstaben, Zahlen oder Symbole, oder andererseits Muster. Während es sich bei den "Sehzeichen" jeweils um ein einzelnes feststehendes Zeichen handelt, das in seinem Proportionen zur Wiedererkennbarkeit durch den Nutzer nur eingeschränkt veränderbar ist, bezeichnet der Begriff des "Musters" eine beliebige graphische Struktur, welche - insbesondere im Gegensatz zu einem Rauschen, das ohne erkennbare Struktur bleibt - über mindestens eine räumliche Periode verfügt, innerhalb der die Struktur des Musters wiederholt dargestellt ist. Anstelle des Begriffs des "Musters" wird daher auch der Begriff des "periodischen Musters" verwendet, um diese Eigenschaft des Musters deutlich zum Ausdruck zu bringen. Im Folgenden sollen diese beiden Begriffe jedoch denselben Begriffsinhalt umfassen.

Aufgrund der elektronischen Ansteuerung kann ein Parameter des auf dem Bildschirm dargestellten Zeichens auf einfache Weise und in einem weiten Rahmen verändert werden. Bei dem "Parameter" handelt es sich um eine Eigenschaft des Zeichens, je nach ausgewähltem Zeichen, insbesondere um eine Ausdehnung, eine Intensität oder eine Farbe (einschließlich schwarz und weiß). Hierbei kann das Zeichen, insbesondere das Muster, mindestens zwei voneinander verschiedene Farben aufweisen, insbesondere um ein chromatische Aberration berücksichtigen zu können. Im Falle des Musters kann eine Struktur wiederholt dargestellt werden, wobei sich durch Wiederholung gleichartige Punkte oder Bereiche über die Struktur des Musters ausbilden können. Bevorzugte Ausgestaltungen gleichartiger Punkte oder Bereiche können bevorzugt als periodische Maxima oder Minima des Musters vorliegen. Während es sich bei den ausgewählten Parametern eines herkömmlichen Sehzeichens, insbesondere einem Buchstaben, einer Zahl oder einem Symbol, also um eine Ausdehnung, insbesondere eine Höhe oder Breite, des Zeichens handeln kann, bezieht sich bei dem periodischen Muster der Parameter bevorzugt auf einen Parameter einer periodischen Funktion, insbesondere eine Wiederholfrequenz. Die "periodische Funktion" bezeichnet hierbei eine Anweisung an eine Ausgestaltung einer zeitlich oder, bevorzugt, räumlich wiederholten Veränderung des Parameters. Die periodische Funktion kann vorzugsweise ausgewählt sein aus einer Sinusfunktion, einer Cosinusfunktion oder einer Überlagerung hiervon. Andere periodische Funktionen sind jedoch denkbar.

Weiterhin kann das Zeichen vorzugsweise entfernungsskaliert auf dem Bildschirm dargestellt werden, das heißt ein Parameter des Zeichens kann in Abhängigkeit von einer Entfernung, aus welcher der Nutzer das Muster betrachtet, gewählt werden. Beispielsweise kann bei einer größeren Entfernung des Nutzers von dem Bildschirm der Parameter entsprechend größer gewählt werden. Umgekehrt kann bei einer geringeren Entfernung des Nutzers von dem Bildschirm der Parameter entsprechend kleiner gewählt werden.

Erfindungsgemäß umfasst der Parameter des auf dem Bildschirm dargestellten Zeichens mindestens eine Ortsfrequenz des periodischen Musters. Der Begriff der "Ortsfrequenz" bezeichnet hierbei einen Kehrwert eines räumlichen Abstands, welcher in der Einheit 1/m oder alternativ als dimensionslose Zahl mit der Einheit "Einheiten pro Grad" oder "Zyklen pro Grad" angegeben werden kann, zwischen zwei benachbart angeordneten gleichartigen Punkten, insbesondere einem Maximum oder einem Minimum, in einer räumlich periodischen Änderung des Musters. Hierbei kann die auf dem Bildschirm dargestellte Ortsfrequenz bevorzugt entsprechend der Entfernung des Bildschirms vom Auge des Nutzers gewählt werden. Hierzu kann die auf dem Bildschirm dargestellte Ortsfrequenz bei einer größeren Entfernung des Nutzers von dem Bildschirm größer und bei einer geringeren Entfernung des Nutzers von dem Bildschirm kleiner gewählt werden. Bevorzugt kann hierbei die Intensität oder die Farbe des Musters entlang einer Richtung der Ausdehnung des Bildschirms dem Verlauf der periodischen Funktion, insbesondere der Sinusfunktion, folgen. Andere Arten der Bestimmung der Ortsfrequenz aus dem Muster sind jedoch denkbar, beispielsweise aus einem Abstand von Punkten gleicher Intensität.

In einer besonders bevorzugten Ausgestaltung der vorliegenden Erfindung kann das periodische Muster als zweidimensionale Überlagerung einer periodischen Funktion, insbesondere der Sinusfunktion, welche sich in eine erste Richtung auf der Ausdehnung des Bildschirms erstreckt, und einer konstanten Funktion, welche sich in eine zweite Richtung auf der Ausdehnung des Bildschirms erstreckt, die vorzugsweise senkrecht zur ersten Richtung angeordnet sein kann, ausgestaltet sein. Der Begriff "senkrecht" bezeichnet hierbei einen Winkel von 90° ± 30°, bevorzugt von 90° ± 15°, besonders bevorzugt von 90° ± 5°, insbesondere von 90° ± 1°. Andere Winkel zwischen der ersten Richtung und der zweiten Richtung sind jedoch ebenfalls möglich. Auf diese Weise kann das Muster in Form von periodisch nebeneinander angeordneten Streifen vorliegen, die auch als "sinusförmiges Gitter" bezeichnet werden können. Andere Arten von Mustern sind jedoch möglich.

In einer weiteren bevorzugten Ausgestaltung kann das Muster derart auf der Ausdehnung des Bildschirms dargestellt werden, dass die erste Richtung oder die zweite Richtung einen festgelegten Winkel in Bezug auf eine Orientierung des Bildschirms einnehmen, wobei der jeweils festgelegte Winkel bevorzugt 0° oder ein Vielfaches von 90° ist. Der Begriff "Orientierung" bezeichnet hierbei eine Richtung, welche parallel zu einem Rand des Bildschirms ist, der üblicherweise die Form eines Rechtecks annimmt. Auf diese Weise kann das Muster an die vorhandene Ausdehnung des Bildschirms angepasst sein. Andere Arten der Darstellung des Musters auf dem Bildschirm sind jedoch denkbar, beispielsweise mit einem in Bezug auf die Orientierung des Bildschirms festgelegten Winkel von 45° oder einem ungeraden Vielfachen davon.

In einer weiteren bevorzugten Ausgestaltung kann die ausgewählte periodische Funktion mit einer weiteren Funktion überlagert werden, vorzugsweise mit einer zunehmenden Funktion oder abnehmenden Funktion. Auf diese Weise kann die Amplitude der ausgewählten periodischen Funktion entlang der Richtung der Ausdehnung des Bildschirms zunehmen oder abnehmen. Besonders bevorzugt ist es jedoch wenn, alternativ oder zusätzlich hierzu, die Ortsfrequenz des periodischen Musters entlang der Richtung der Ausdehnung des Bildschirms zunimmt oder abnimmt. Auf diese Weise kann die Darstellung des Musters auf dem Bildschirm bereits eine Reihe von Ortsfrequenzen aufweisen, die vorzugweise in aufsteigender Reihe oder in abfallender Reihe entlang der Richtung der Ausdehnung des Bildschirms angeordnet sind.

Wie unten näher erläutert, kann alternativ oder zusätzlich, das periodische Muster zunächst in einer ersten Richtung und anschließend in einer zweiten Richtung, die senkrecht zur ersten Richtung angeordnet ist, dargestellt werden. Auf diese Weise können für das sphärozylindrische Brillenglas mit astigmatischer Wirkung nacheinander die Scheitelbrechwerte jedes der beiden Hauptschnitte, die senkrecht zueinander stehen, ermittelt werden.

Gemäß Schritt b) erfolgt, vorzugsweise während der Darstellung des Zeichens auf dem Bildschirm gemäß Schritt a), in Abhängigkeit von dem auf dem Bildschirm dargestellten Zeichen eine Erfassung einer Reaktion des Nutzers. Der Begriff "Reaktion" bezeichnet eine Antwort des Nutzers auf einen Stimulus des Auges in Folge der Darstellung des Zeichens auf dem Bildschirm. Der Begriff der "Erfassung" bezieht sich hierbei auf eine Aufnahme eines Messsignals, das der Nutzer in Folge seiner einer Reaktion erzeugt. Insbesondere kann die Erfassung der Reaktion des Nutzers während Schritt b) monokular erfolgen, d.h. die Reaktion des Nutzers wird einzeln, bevorzugt nacheinander, für jedes der beiden Augen des Nutzers erfasst. Hierzu kann der Nutzer bevorzugt das jeweils andere, nicht verwendete Auge abdecken. Ein Wechsel des jeweiligen Auges zur Betrachtung des Zeichens auf dem Bildschirm kann hierbei beispielsweise durch eine entsprechende Menüführung mittels des mobilen Kommunikationsgeräts veranlasst werden.

In einer besonders bevorzugten Ausgestaltung kann eine Eingabeeinheit vorgesehen sein, die zur Erfassung der Reaktion des Nutzers in Abhängigkeit von dem auf dem Bildschirm dargestellten Zeichen eingerichtet ist. Bei der Eingabeeinheit kann es sich um eine Tastatur, insbesondere um eine Tastatur, die über Tasten verfügt, die der Nutzer bedienen, bevorzugt drücken, kann, handeln. Vorzugsweise kann es sich, alternativ oder zusätzlich, um eine auf einem berührungsempfindlichen Bildschirm (Touchscreen) des mobilen Kommunikationsgeräts dargestellte virtuelle Tastatur handeln, die der Nutzer ebenfalls bedienen, bevorzugt drücken, kann. Durch ein Bedienen der Eingabeeinheit kann somit der Nutzer mittels der Eingabeeinheit ein Messsignal erzeugen, das an eine unten näher beschriebene Auswerteeinheit weitergeleitet werden kann.

Gemäß Schritt c) erfolgt, vorzugsweise während der Ausführung von Schritt b), eine Feststellung eines Zeitpunkts, der dadurch festgelegt ist, dass sich zu dem festgestellten Zeitpunkt aus der Reaktion des Nutzers eine Erkennbarkeit des auf dem Bildschirm dargestellten Zeichens für den Nutzer ergibt. Der Begriff der "Erkennbarkeit" umfasst hierbei, dass der Nutzer das auf dem Bildschirm dargestellte Zeichen gerade noch oder gerade erst erkennen kann. Nimmt die Ortsfrequenz in dem periodischen Muster zunehmend ab, kann hierbei der Zeitpunkt festgestellt werden, bei dem der Nutzer das auf dem Bildschirm dargestellte Zeichen gerade erkennen kann. Umgekehrt kann, falls die Ortsfrequenz in dem periodischen Muster zunehmend anwächst, hierbei der Zeitpunkt festgestellt werden, an dem der Nutzer das auf dem Bildschirm dargestellte Zeichen gerade noch erkennen kann. Um den Nutzer dazu zu bewegen, die Reaktion in der jeweils gewünschten Weise vorzunehmen, kann ein Teil des Bildschirms oder, alternativ oder zusätzlich, eine akustische Ausgabeeinheit dazu verwendet werden, um den Nutzer entsprechend zu informieren oder zur gewünschten Reaktion anzuhalten. Hierbei kann die Auswerteeinheit aus dem während Schritt b) durch das Bedienen der Eingabeeinheit erzeugten Messsignals, das die Auswerteeinheit weitergeleitet wurde, den gewünschten Zeitpunkt, zu dem sich aus der Reaktion des Nutzers ergibt, dass eine Erkennbarkeit der auf dem Bildschirm dargestellten Ortsfrequenz für den Nutzer gegeben ist, feststellen.

Gemäß Schritt d) erfolgt, bevorzugt in der Auswerteeinheit, vorzugsweise im Anschluss an die Feststellung des Zeitpunkts entsprechend Schritt c), eine Bestimmung eines Wertes für den Refraktionsfehler des Auges des Nutzers aus dem Wert des Parameters, der zu dem festgestellten Zeitpunkt dazu verwendet wurde, den ausgewählten Parameter des Zeichens auf dem Bildschirm einzustellen. Gemäß der vorliegenden Erfindung wird der Wert für den Refraktionsfehler aus der zu dem Zeitpunkt festgelegten Ortsfrequenz des Musters bestimmt, zu welcher der Nutzer gemäß Schritt c) angegeben hat, dass er die auf dem Bildschirm gerade noch oder gerade erst erkennen kann. In vorteilhafter Weise kann der Nutzer das hier vorgeschlagene Verfahren selbst subjektiv durchführen. Er ist hierbei weder auf eine ortsfest installierte Vorrichtung noch auf einen Bediener, insbesondere einen Augenoptiker, angewiesen.

Zur Bestimmung des Wertes für den Refraktionsfehler aus der zu dem festgestellten Zeitpunkt festgelegten Ortsfrequenz des Musters kann eine Ermittlung einer sogenannten "Scheinauflösung" des Nutzers erfolgen. Die Scheinauflösung betrifft ein physikalisches Phänomen, das eine Kontrastübertragung an einer Auflösungsgrenze eines defokussierten optischen Systems beschreibt. Die Scheinauflösung eines optischen System wird gemäß M. Young, Optik, Laser, Wellenleiter, 1997, in Bezug auf die Phase einer optischen Übertragungsfunktion (OTF), die im Allgemeinen ungleich Null ist, festgelegt. Eine von Null verschiedene Phase weist auf eine räumliche Verschiebung des Musters gegenüber der durch die geometrische Optik vorausgesagten Position des Musters hin. Nimmt die Phase der OTF einen Wert von π an, so ist das Bild eines sinusförmigen Gitters gegenüber dem geometrisch-optischen Bild um eine halbe Periode verschoben. Dieses Phänomen tritt insbesondere dann auf, wenn Details unterhalb der Auflösungsgrenze eines defokussierten optischen Systems liegen. Erfindungsgemäß wird das physikalische Phänomen der Scheinauflösung nun dazu verwendet, die Auflösungsgrenze des Auges des Nutzers, bei der dieses Phänomen auftritt, zu bestimmen und hieraus die Defokussierung zu berechnen, die der gewünschten Korrektion des Refraktionsfehlers des Auges des Nutzers entspricht.

Im Allgemeinen kann die Scheinauflösung durch eine Bessel-Funktion beschrieben werden. Nach F. Schaeffel und A. de Queiroz, Alternative Mechanisms of Enhanced Underwater Vision in the Gartner Snakes Tamnophis melanogaster and T. couchii, Copeia 1990 (1), S. 50-58, kann jedoch für die Ortsfrequenz, die der Scheinauflösung entspricht, folgende Näherung gemäß Gleichung (1)

$$Ortsfrequenz = \frac{21,3}{Defokus\,[D] \cdot Pupillendurchmesser\,[m]} \quad (1)$$

angegeben werden, wobei hier die Ortsfrequenz als dimensionslose Zahl "Einheiten pro Grad" oder "Zyklen pro Grad" angegeben wird. Durch Umformen kann hieraus die Defokussierung in Dioptrien D gemäß Gleichung (2)

$$Defokus\,[D] = \frac{21,3}{Ortsfrequenz \cdot Pupillendurchmesser\,[m]} \quad (2)$$

ermittelt werden, die in erster Näherung dem sphärischen Äquivalent der Korrektion entspricht.

Da gemäß Gleichung (1) und (2) sowohl die Scheinauflösung als auch die Defokussierung von einem Durchmesser der Pupille (Pupillendurchmesser) im Auge des Nutzers abhängig sind, wird der Pupillendurchmesser bei der Bestimmung des Refraktionsfehlers des Auges des Nutzers verwendet. Hierzu kann der Pupillendurchmesser auf einen Wert von 2 bis 3 mm geschätzt werden, wobei dieser Wert einem durchschnittlichen Durchmesser der Pupille bei Tag entspricht. Die "Pupille" bezeichnet hierbei eine in jedem Auge vorhandene Eintrittsöffnung, durch welche Strahlung in Form von Licht in das Innere des Auges eintreten kann. In umgekehrter Richtung kann die Pupille als Austrittsöffnung betrachtet werden, durch welche eine Blickrichtung des Nutzers aus dem Auge auf die Umgebung festgelegt werden kann.

Vorzugsweise kann der Pupillendurchmesser jedoch messtechnisch erfasst werden. Hierzu kann vor oder nach, bevorzugt jedoch während einem der Schritte a) bis c), insbesondere während Schritt a), ein Bild einer Augenpartie des Nutzers aufgenommen werden. Hierzu kann bevorzugt eine Kamera verwendet werden, wobei die Kamera vorzugsweise von dem mobilen Kommunikationsgerät umfasst sein kann. Hierbei kann es sich um eine Rückkamera oder bevorzugt um eine Frontkamera des mobilen Kommunikationsgeräts handeln. Auf diese Weise kann bevorzugt das gewünschte Bild der Augenpartie des Nutzers mittels der Kamera an jedem beliebigen Ort aufgenommen werden. Insbesondere mittels Bildverarbeitung, können, vorzugsweise in der Auswerteeinheit, aus dem aufgenommenen Bild geometrische Daten der Pupille, insbesondere Lage und Durchmesser der Pupille, ermittelt werden. Andere Arten der Bestimmung des Pupillendurchmessers sind jedoch ebenfalls möglich.

In einer weiteren Ausgestaltung der vorliegenden Erfindung kann bei bekanntem Abstand zwischen der Kamera und dem Auge des Nutzers somit eine "Pupillendistanz" ermittelt werden, wobei die Pupillendistanz anschließend auf unterschiedliche Entfernungen korrigiert werden kann. Eine Bestimmung dieses Abstands kann bevorzugt durch eine Abstandsmessung erfolgen, insbesondere mittels einer Abstandsmessung, über welche das mobile Kommunikationsgerät bereits verfügen kann. Alternativ oder zusätzlich kann dieser Abstand durch Triangulation über eine bekannte Pixelanzahl der Kamera bei Detektion eines bekannten Gegenstandes oder Bildinhaltes bestimmt werden. Darüber hinaus können durch eine Auswertung von Fotos oder Videos, die mittels Sensoren des mobilen Kommunikationsgeräts aufgenommen wurden, unter Einsatz von maschinellem Lernen, vorzugsweise in der Auswerteeinheit, ein oder mehrere Trageparameter des Nutzers, bevorzugt der Hornhautscheitelabstand zu dem Auge des Nutzers oder der Pupillenabstand zwischen den beiden Augen des Nutzers, bestimmt werden.

Wie bereits erwähnt, kann durch die Ermittlung der Scheinauflösung in erster Näherung das sphärische Äquivalent der Korrektion bestimmt werden. Wird die Scheinauflösung in mindestens zwei Meridianen bestimmt, vorzugsweise durch eine oben oder unten näher beschriebene Darstellung des periodischen Musters zunächst in einer ersten Richtung und anschließend in einer zweiten Richtung, die senkrecht zur ersten Richtung angeordnet ist, kann hierdurch die Bestimmung der sphärozylindrischen Korrektion erfolgen. Für weitere Einzelheiten hierzu wird auf WO 2018/077690 A1 verwiesen.

In einer weiteren Ausgestaltung der vorliegenden Erfindung kann das auf dem Bildschirm dargestellte Muster ein monochromatisches Muster sein. In dieser Ausgestaltung kann bei der Messung der Scheinauflösung zusätzlich eine chromatische Aberration berücksichtigt werden. Insbesondere durch longitudinale chromatische Aberration kommt es dazu, dass bei einem rechtsichtigen oder durch Korrektion rechtsichtiges Auge, monochromatisches Licht mit einer Wellenlänge von etwa 450 nm vor der Netzhaut gebündelt wird, während monochromatisches Licht einer Wellenlänge größer als 600 nm hinter der Netzhaut gebündelt wird. Durch diese natürlich auftretende Defokussierung kann es für den Nutzer vorteilhaft sein, seine Scheinauflösung bevorzugt für beide Wellenlängen nacheinander auf dem Bildschirm einzustellen. Alternativ kann der Nutzer eine mittlere Scheinauflösung bei gleichzeitiger Darstellung der beiden monochromatischen Wellenlängen einstellen.

Da, physikalisch betrachtet, die Defokussierung unabhängig von ihrem Vorzeichen zu demselben Wert für die Scheinauflösung führt, kann in einem weiteren Verfahrensschritt unterschieden werden, ob eine Kurzsichtigkeit oder eine Weitsichtigkeit des Nutzers vorliegt. In einer ersten Ausgestaltung kann dies durch eine einfache Frageprozedur erfolgen, zum Beispiel indem der Nutzer zu einer Eingabe auf eine Frage wie "Sehen Sie in der Ferne schlecht?", die ihm vorzugsweise mittels des mobilen Kommunikationsgeräts gestellt werden kann, mit den beiden Antwortmöglichkeiten "Ja" und "Nein" aufgefordert wird. Alternativ oder zusätzlich kann in einer weiteren Ausgestaltung ein optischer Stimulus auf dem mobilen Kommunikationsgerät bereitgestellt und der Nutzer zu einer Eingabe aufgefordert werden. Sieht der Nutzer den Stimulus auf die kurze Distanz nicht, kann davon ausgegangen werden, dass er kurzsichtig ist. Besonders vorteilhaft ist hierbei der hier vorgeschlagenen Ausgestaltungen.

In einer weiteren Ausgestaltung kann berücksichtigt werden, dass der Nutzer eine Brille trägt und das Auge des Nutzers einen hohen Wert für die Scheinauflösung aufweist. In diesem Falle kann durch eine weitere Frageprozedur eine weitere Rückmeldung des Nutzers erfragt werden, ob seine Brille noch in Ordnung sei.

Alternativ kann in einer weiteren Ausgestaltung, in welcher die Scheinauflösung verringert ist, der Nutzer dazu angehalten werden, zurückmelden, dass er eine neue Brille brauche.

Insgesamt wird im Rahmen der vorliegenden Erfindung zur Bestimmung der Scheinauflösung ein so genannter "psychophysischer Algorithmus" eingesetzt. Der psychophysische Algorithmus bezeichnet hierbei eine Vorgehensweise, welche auf gesetzmäßigen Wechselbeziehungen zwischen einem subjektivem mentalem Erleben des Nutzers und quantitativ messbaren, objektiven physikalischen Stimuli als den Auslöser für das Erleben des Nutzers basiert. Das Erleben des Nutzers besteht hierbei in der konkreten Erfahrung, dass er ein ausgewähltes Muster gerade nicht mehr oder erstmals erkennen kann. Hierbei wurde das Muster durch Verwendung eines objektiven Parameters auf einem Bildschirm, den der Nutzer betrachtet, dargestellt. Reagiert der Nutzer gemäß der an ihn gestellten Aufforderung auf diese Erfahrung, indem er eine entsprechende Eingabe an eine Vorrichtung abgibt, so kann aus der Eingabe unter Kenntnis des objektiven Parameters in Bezug auf das Muster, eine quantitativ messbare Größe bestimmt werden, aus der sich im Falle der vorliegenden Erfindung die Scheinauflösung eines Auges eines Nutzers und hieraus der damit in Beziehung stehende Refraktionsfehler des Auges des Nutzers als objektive physikalische Größe bestimmen lässt.

In einem weiteren Aspekt betrifft die vorliegende Erfindung ein Computerprogramm zur Bestimmung eines Refraktionsfehlers eines Auges eines Nutzers, wobei das Computerprogramm dazu eingerichtet ist, die Bestimmung des Refraktionsfehlers des Auges des Nutzers gemäß dem hierin beschriebenen Verfahren zur Bestimmung eines Refraktionsfehlers eines Auges eines Nutzers durchzuführen.

In einem weiteren Aspekt betrifft die vorliegende Erfindung ein Verfahren zur Herstellung eines Brillenglases, wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich des Refraktionsfehlers des Auges des Nutzers umfassen, wobei eine Bestimmung des Refraktionsfehlers des Auges des Nutzers gemäß dem hierin beschriebenen Verfahren zur Bestimmung eines Refraktionsfehlers eines Auges eines Nutzers erfolgt. Darüber hinaus umfassen die Zentrierdaten auch weitere Daten, die sich auf eine ausgewählte Brille beziehen. Beispielsweise bezieht sich die Pupillendistanz individuell auf den Nutzer, während ein Durchblickpunkt durch ein Zusammenspiel mit der Brillenfassung festgelegt wird.

In einem weiteren Aspekt betrifft die vorliegende Erfindung eine Vorrichtung zur Bestimmung des Refraktionsfehlers des Auges des Nutzers. Erfindungsgemäß umfasst die Vorrichtung

- einen Bildschirm, der zur Darstellung eines Zeichen und einer Veränderung eines Parameters des Zeichens eingerichtet ist;
- eine Eingabeeinheit, die zur Erfassung einer Reaktion des Nutzers in Abhängigkeit von dem auf dem Bildschirm dargestellten Zeichen eingerichtet ist; und
- eine Auswerteeinheit, die zur Feststellung eines Zeitpunkts, zu dem sich aus der Reaktion des Nutzers eine Erkennbarkeit des auf dem Bildschirm dargestellten Zeichens für den Nutzer ergibt, und zur Bestimmung eines Wertes für den Refraktionsfehler des Auges des Nutzers aus dem zu dem Zeitpunkt festgelegten Parameter eingerichtet ist,

wobei der Bildschirm zur Darstellung eines periodischen Musters als das Zeichen eingerichtet ist, wobei der Parameter des auf dem Bildschirm dargestellten Zeichens mindestens eine Ortsfrequenz des periodischen Musters umfasst, und wobei die Auswerteeinheit zur Bestimmung des Wertes für den Refraktionsfehler des Auges des Nutzers aus der zu dem Zeitpunkt festgelegten Ortsfrequenz des periodischen Musters eingerichtet ist.

In einer besonders bevorzugten Ausgestaltung kann die Vorrichtung weiterhin eine Kamera umfassen, wobei die Kamera dazu eingerichtet ist, eine Aufnahme eines Bildes des Auges des Nutzers vorzunehmen. In dieser Ausgestaltung kann die Auswerteeinheit weiterhin dazu eingerichtet sein, durch Bildverarbeitung dieses Bildes und durch Bestimmung einer Pupillendistanz zwischen der Kamera und dem Auge des Nutzers eine Ermittlung des Pupillendurchmessers des Auges des Nutzers auszuführen.

Für Definitionen und optionale Ausgestaltungen des Computerprogramms und der Vorrichtung zur Bestimmung eines Refraktionsfehlers eines Auges eines Nutzers sowie des Verfahrens zur Herstellung eines Brillenglases wird auf die oben oder unten stehende Beschreibung des Verfahrens zur Bestimmung eines Refraktionsfehlers eines Auges eines Nutzers verwiesen.

Die erfindungsgemäße Vorrichtung und die vorliegenden Verfahren weisen gegenüber herkömmlichen Vorrichtungen und Verfahren zahlreiche Vorteile auf. Damit kann eine subjektive Ermittlung der Korrektion eines Refraktionsfehlers eines Auges eines Nutzers ohne Spezialgeräte erfolgen und insbesondere auch von einem Laien benutzt werden. Weiterhin wird hierbei in vorteilhafter Weise das physikalische Phänomen der Scheinauflösung für die Ermittlung der Korrektion verwendet, das es erlaubt, die Defokussierung des Auges des Nutzers auf einfache Weise zu bestimmen.

Zusammenfassend sind im Rahmen der vorliegenden Erfindung folgende Ausführungsformen besonders bevorzugt:

Ausführungsform 1. Verfahren zur Bestimmung eines Refraktionsfehlers eines Auges eines Nutzers, wobei das Verfahren die folgenden Schritte umfasst:

a) Darstellen eines Zeichens auf einem Bildschirm, wobei ein Parameter des auf dem Bildschirm dargestellten Zeichens verändert wird;
b) Erfassen einer Reaktion des Nutzers in Abhängigkeit von dem auf dem Bildschirm dargestellten Zeichen;
c) Feststellen eines Zeitpunkts, zu dem sich aus der Reaktion des Nutzers eine Erkennbarkeit des auf dem Bildschirm dargestellten Zeichens für den Nutzer ergibt; und
d) Bestimmen eines Wertes für den Refraktionsfehler des Auges des Nutzers aus dem zu dem Zeitpunkt festgelegten Parameter,

wobei das auf dem Bildschirm dargestellte Zeichen ein periodisches Muster ist, wobei der Parameter des auf dem Bildschirm dargestellten Musters mindestens eine Ortsfrequenz umfasst, und wobei der Wert für den Refraktionsfehler aus der zu dem Zeitpunkt festgelegten Ortsfrequenz des Musters bestimmt wird.

Ausführungsform 2. Verfahren nach der vorangehenden Ausführungsform, wobei die Ortsfrequenz des Musters entfernungsskaliert auf dem Bildschirm dargestellt wird.

Ausführungsform 3. Verfahren nach einer der beiden vorangehenden Ausführungsformen, wobei die Ortsfrequenz des auf dem Bildschirm dargestellten Musters vergrößert oder verkleinert wird.

Ausführungsform 4. Verfahren nach der vorangehenden Ausführungsform, wobei die Ortsfrequenz des Musters des auf dem Bildschirm dargestellten Musters zeitlich oder räumlich verändert wird.

Ausführungsform 5. Verfahren nach einer der vorangehenden Ausführungsformen, wobei das Muster aus einer Überlagerung einer periodischen Funktion und einer konstanten Funktion gebildet wird.

Ausführungsform 6. Verfahren nach der vorangehenden Ausführungsform, wobei die periodische Funktion ausgewählt ist aus einer Sinusfunktion, einer Cosinusfunktion oder einer Überlagerung hiervon.

Ausführungsform 7. Verfahren nach einer der vorangehenden Ausführungsformen, wobei die periodische Funktion zusätzlich derart mit einer zunehmenden Funktion oder einer abnehmenden Funktion überlagert wird, dass die Ortsfrequenz des Musters in einer Richtung zunimmt oder abnimmt.

Ausführungsform 8. Verfahren nach der vorangehenden Ausführungsform, wobei die Richtung einen Winkel in Bezug auf eine Orientierung des Bildschirms einnimmt, wobei der Winkel 0° oder ein Vielfaches von 90° ist.

Ausführungsform 9. Verfahren nach einer der vorangehenden Ausführungsformen, wobei das Muster zunächst in einer ersten Richtung dargestellt wird und anschließend in einer gegen die erste Richtung veränderten zweiten Richtung dargestellt wird. Ausführungsform 10. Verfahren nach der vorangehenden Ausführungsform, wobei die jeweilige Ortsfrequenz des Musters in die erste Richtung und in die zweite Richtung zu einer Bestimmung einer sphärozylindrischen Korrektur verwendet wird.

Ausführungsform 11. Verfahren nach einer der vorangehenden Ausführungsformen, wobei der Wert (220) des Refraktionsfehlers des Auges (112) des Nutzers (114) einer Defokussierung des Auges (112) des Nutzers (114) entspricht, wobei die Defokussierung als *Defokus* [*D*] in Dioptrien *D* gemäß Gleichung (2)

$$Defokus\ [D]\ =\ \frac{21{,}3/m}{Ortsfrequenz \cdot Pupillendurchmesser\ [m]} \qquad (2)$$

bestimmt wird, wobei die Ortsfrequenz, welche der Nutzer (114) gerade noch oder gerade erst erkennen kann, als dimensionslose Zahl angegeben wird, und wobei das Auge (112) des Nutzers (114) den Pupillendurchmesser (156) in m aufweist.

Ausführungsform 12. Verfahren nach einer der vorangehenden Ausführungsformen, wobei der Pupillendurchmesser des Auges des Nutzers auf einen Wert von 2 mm bis 3 mm geschätzt wird.

Ausführungsform 13. Verfahren nach einer der beiden vorangehenden Ausführungsformen, wobei der Pupillendurchmesser des Auges des Nutzers messtechnisch erfasst wird

Ausführungsform 14. Verfahren nach einer der vorangehenden Ausführungsformen, wobei der Pupillendurchmesser des Auges des Nutzers durch Aufnahme eines Bildes des Auges des Nutzers mittels einer Kamera, durch Bildverarbeitung des Bildes und durch Bestimmung einer Pupillendistanz zwischen der Kamera und dem Auge des Nutzers ermittelt wird.

Ausführungsform 15. Verfahren nach einer der vorangehenden Ausführungsformen, wobei die Erfassung der Reaktion des Nutzers während Schritt b) monokular erfolgt, wobei die Reaktion des Nutzers einzeln, bevorzugt nacheinander, für jedes der beiden Augen des Nutzers erfasst wird, wobei der Nutzer vorzugsweise das jeweils andere, nicht verwendete Auge abdeckt.

Ausführungsform 16. Computerprogramm zur Bestimmung eines Refraktionsfehlers eines Auges eines Nutzers, wobei das Computerprogramm dazu eingerichtet ist, die Verfahrensschritte nach einer der vorangehenden Ausführungsformen durchzuführen. Ausführungsform 17. Verfahren zur Herstellung eines Brillenglases, wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich des Refraktionsfehlers des Auges des Nutzers umfassen, wobei eine Bestimmung des Refraktionsfehlers des Auges des Nutzers gemäß den Verfahrensschritten nach einer der vorangehenden Ausführungsformen in Bezug auf das Verfahren zur Bestimmung eines Refraktionsfehlers eines Auges eines Nutzers erfolgt.

Ausführungsform 18. Vorrichtung zur Bestimmung des Refraktionsfehlers des Auges des Nutzers, wobei die Vorrichtung umfasst:

- einen Bildschirm, der zur Darstellung eines Zeichen und einer Veränderung eines Parameters des Zeichens eingerichtet ist;
- eine Eingabeeinheit, die zur Erfassung einer Reaktion des Nutzers in Abhängigkeit von dem auf dem Bildschirm dargestellten Zeichen eingerichtet ist; und
- eine Auswerteeinheit, die zur Feststellung eines Zeitpunkts, zu dem sich aus der Reaktion des Nutzers eine Erkennbarkeit des auf dem Bildschirm (dargestellten Zeichens für den Nutzer ergibt, und zur Bestimmung eines Wertes für den Refraktionsfehler des Auges des Nutzers aus dem zu dem Zeitpunkt festgelegten Parameter eingerichtet ist,

wobei der Bildschirm zur Darstellung eines periodischen Musters als das Zeichen eingerichtet ist, wobei der Parameter des auf dem Bildschirm dargestellten Zeichens mindestens eine Ortsfrequenz des periodischen Musters umfasst, und wobei die Auswerteeinheit zur Bestimmung des Wertes für den Refraktionsfehler des Auges des Nutzers aus der zu dem Zeitpunkt festgelegten Ortsfrequenz des periodischen Musters eingerichtet ist.

Ausführungsform 19. Vorrichtung nach der vorangehenden Ausführungsform, wobei die Vorrichtung weiterhin eine Kamera umfasst, wobei die Kamera dazu eingerichtet ist, eine Aufnahme eines Bildes des Auges des Nutzers vorzunehmen.

Ausführungsform 20. Vorrichtung nach der vorangehenden Ausführungsform, wobei die Auswerteeinheit weiterhin dazu eingerichtet ist, durch Bildverarbeitung des Bildes des Auges des Nutzers und durch Bestimmung einer Pupillendistanz zwischen der Kamera und dem Auge des Nutzers eine Ermittlung des Pupillendurchmessers des Auges des Nutzers auszuführen.

Ausführungsform 21. Vorrichtung nach einer der drei vorangehenden Ausführungsformen, wobei die Vorrichtung als mobiles Kommunikationsgerät ausgestaltet ist, wobei das mobile Kommunikationsgerät den Bildschirm, die Eingabeeinheit, die Auswerteeinheit und optional die Kamera umfasst

Ausführungsform 22. Vorrichtung nach der vorangehenden Ausführungsform, wobei das mobile Kommunikationsgerät als Smartphone ausgestaltet ist.

In einem weiteren Aspekt können das vorstehend beschriebene Verfahren und/oder die vorstehend beschriebene Vorrichtung und/oder das vorstehend beschriebene Computerprogramm zusammen mit wenigstens einem weiteren Verfahren und/oder wenigstens einer weiteren Vorrichtung und/oder einem weiteren Computerprogramm zur Anwendung kommen. Bei diesem wenigstens einen weiteren Verfahren kann es sich um ein beispielsweise um ein Verfahren zur Bestimmung eines Refraktionsfehlers eines Auges eines Nutzers handeln, wobei dieses Verfahren die folgenden Schritte umfasst:

a) Darstellen eines Zeichens auf einem Bildschirm, wobei ein Parameter des auf dem Bildschirm dargestellten Zeichens verändert wird;
b) Erfassen einer Augenbewegungsmetrik des Auges des Nutzers in Abhängigkeit von dem auf dem Bildschirm dargestellten Zeichen; und
c) Feststellen eines Zeitpunkts, zu dem sich aus der Augenbewegungsmetrik des Auges des Nutzers eine Erkennungsschwelle des Nutzers für das auf dem Bildschirm dargestellte Zeichen ergibt; und
d) Bestimmen eines Wertes für den Refraktionsfehler des Auges des Nutzers aus dem zu dem Zeitpunkt festgelegten Parameter.

Alternativ oder zusätzlich zu dem vorstehend beschriebenen Verfahren kann es sich bei dem wenigstens einen weiteren Verfahren beispielsweise auch um ein Verfahren zur Bestimmung mindestens eines optischen Parameters eines Brillenglases handeln, wobei dieses Verfahren folgende Schritte umfasst:

a) Aufnehmen eines Bildes unter Verwendung eines Brillenglases; und
b) Ermitteln mindestens eines optischen Parameters des Brillenglases mittels Bildverarbeitung des Bildes, wobei das Bild eine die Augen einschließende Augenpartie und/oder eine an die Augen angrenzende Gesichtspartie eines Nutzers des Brillenglases umfasst.

Alternativ oder zusätzlich zu den vorstehend beschriebenen Verfahren kann es sich bei dem wenigstens einen weiteren Verfahren beispielsweise auch um ein Verfahren zum Vermessen der Brechkraftverteilung eines linken und/oder eines rechten Brillenglases in einer Brillenfassung handeln, in welchem in einem ersten Schritt mittels wenigstens einer Bilderfassungseinrichtung wenigstens eine erste Abbildung einer Szene aus wenigstens einer ersten Aufnahmeposition erfasst wird, wobei diese wenigstens eine erste Abbildung wenigstens zwei Strukturpunkte aufweist und ein linkes und/oder ein rechtes Brillenglas in einer Brillenfassung mit einem ein Koordinatensystem der Brillenfassung definierenden Abschnitt der Brillenfassung enthält, wobei der wenigstens eine Abbildungsstrahlengang für jeden dieser wenigstens zwei Strukturpunkte das erste und/oder das zweite Brillenglas der Brillenfassung jeweils mindestens einmal passiert und mindestens einmal nicht passiert. Jeder Abbildungsstrahlengang umfasst die Position des Strukturpunkts sowie den in die wenigstens eine Bilderfassungseinrichtung einfallenden Hauptstrahl. In einem weiteren Schritt, der zeitlich vor oder nach dem ersten Schritt liegen kann, wird wenigstens eine weitere Abbildung der Szene ohne das erste und/oder das zweite Brillenglas der Brillenfassung oder ohne die Brillenfassung enthaltend das erste und/oder das zweite Brillenglas mit denselben wenigstens zwei Strukturpunkten der ersten Abbildung einer Szene mittels wenigstens einer Bilderfassungseinrichtung aus der ersten Aufnahmeposition oder aus wenigstens einer von der ersten Aufnahmeposition verschiedenen weiteren Aufnahmeposition erfasst. Die wenigstens eine Bilderfassungseinrichtung kann in dem weiteren Schritt identisch oder verschieden zu der wenigstens einen Bilderfassungseinrichtung aus dem ersten Schritt sein. Bevorzugt ist die wenigstens eine Bilderfassungseinrichtung in dem weiteren Schritt identisch zu der wenigstens einen Bilderfassungseinrichtung aus dem ersten Schritt. Darauf werden in einem Schritt des Berechnens die Koordinaten dieser wenigstens zwei Strukturpunkte in einem zu dem Koordinatensystem der Brillenfassung referenzierten Koordinatensystem der Abbildung dieser Szene aus dem jeweiligen wenigstens einen Strahlengang dieser wenigstens zwei Strukturpunkte, welcher das linke und/oder rechte Brillenglas jeweils nicht passiert hat, und der wenigstens einen weiteren Abbildung der Szene mittels Bildauswertung bestimmt. Im Anschluss daran wird die Brechkraftverteilung in einem Schritt des Bestimmens einer Brechkraftverteilung für wenigstens einen Abschnitt des linken Brillenglases in dem Koordinatensystem der Brillenfassung und/oder in einem Schritt des Bestimmens einer Brechkraftverteilung für wenigstens einen Abschnitt des rechten Brillenglases in dem Koordinatensystem der Brillenfassung, jeweils aus den Abbildungsstrahlungsgängen, welche das jeweilige Brillenglas passiert haben, bestimmt.

Alternativ oder zusätzlich zu den vorstehend beschriebenen Verfahren kann es sich bei dem wenigstens einen weiteren Verfahren beispielsweise auch um ein Verfahren zum Vermessen der Brechkraftverteilung eines linken und/oder eines rechten Brillenglases in einer Brillenfassung handeln, in welchem in einem ersten Schritt mittels wenigstens einer Bilderfassungseinrichtung wenigstens eine erste Abbildung einer Szene aus wenigstens einer ersten Aufnahmeposition erfasst wird, wobei diese wenigstens eine erste Abbildung wenigstens zwei Strukturpunkte aufweist und ein linkes und/oder ein rechtes Brillenglas in einer Brillenfassung mit einem ein Koordinatensystem der Brillenfassung definierenden Abschnitt der Brillenfassung enthält, wobei der wenigstens eine Abbildungsstrahlengang für jeden dieser wenigstens zwei Strukturpunkte das erste und/oder das zweite Brillenglas der Brillenfassung jeweils mindestens einmal passiert und mindestens einmal nicht passiert. Jeder Abbildungsstrahlengang umfasst die Position des Strukturpunkts sowie den in die wenigstens eine Bilderfassungseinrichtung einfallenden Hauptstrahl. In einem weiteren Schritt, der zeitlich vor oder nach dem ersten Schritt liegen oder gleichzeitig mit dem ersten Schritt erfolgen kann, wird wenigstens eine weitere Abbildung der Szene mit dem linken und/oder dem rechten Brillenglas in einer Brillenfassung und mit einem ein Koordinatensystem der Brillenfassung definierenden Abschnitt der Brillenfassung mittels wenigstens einer Bilderfassungseinrichtung aus wenigstens einer von der ersten Aufnahmeposition verschiedenen weiteren Aufnahmeposition mit wenigstens einem Abbildungsstrahlengang für dieselben wenigstens zwei in der ersten Abbildung erfassten Strukturpunkte erfasst, wobei dieser wenigstens eine Abbildungsstrahlengang das erste und/oder das zweite Brillenglas der Brillenfassung jeweils mindestens einmal passiert und mindestens einmal nicht passiert. Darauf werden in einem weiteren Schritt die Koordinaten der wenigstens zwei Strukturpunkte in einem zu dem Koordinatensystem der Brillenfassung referenzierten Koordinatensystem der Szene aus dem jeweiligen wenigstens einen Strahlengang dieser wenigstens zwei Strukturpunkte, welcher das linke und/oder rechte Brillenglas jeweils nicht passiert hat und der wenigstens einen weiteren Abbildung der Szene mittels Bildauswertung berechnet. Anschließend wird die Brechkraftverteilung für wenigstens einen Abschnitt des linken Brillenglases in dem Koordinatensystem der Brillenfassung berechnet und/oder es wird die Brechkraftverteilung für wenigstens einen Abschnitt des rechten Brillenglases in dem Koordinatensystem der Brillenfassung, jeweils aus den Abbildungsstrahlengängen, welche das jeweilige Brillenglas passiert haben, bestimmt.

Bevorzugt wird in den beiden vorstehenden Verfahren zum Vermessen der Brechkraftverteilung eines linken und/oder eines rechten Brillenglases, vorzugsweise in einer Brillenfassung, eine Vielzahl von Strukturpunkten in der jeweils ersten Abbildung einer Szene aus jeweils wenigstens einer ersten Aufnahmeposition erfasst und die jeweils darauf folgenden Schritte anhand dieser jeweiligen Vielzahl von Strukturpunkten vorgenommen. Unter einer Vielzahl von Strukturpunkten werden bevorzugt wenigstens 10, weiter bevorzugt wenigstens 100, besonders bevorzugt wenigstens 1000 und ganz besonders bevorzugt wenigstens 10000 Strukturpunkte verstanden. Insbesondere ist eine Vielzahl von Strukturpunkten ≥100 Strukturpunkte und ≤1000 Strukturpunkte.

Alternativ oder zusätzlich zu den vorstehend beschriebenen Verfahren kann es sich bei dem wenigstens einen weiteren Verfahren beispielsweise auch um ein Verfahren zur Bestimmung der Brechkraftverteilung eines Brillenglases handeln, welches aus dem Größen- und/oder Formvergleich der Abbildung des vorderen Augenabschnittes für eine bestimmte Durchblickrichtung eine lokale Brechkraft ermöglicht. Hierzu wird wenigstens eine Aufnahme des vorderen Augenabschnittes mit und ohne davor befindlichem Brillenglas durchgeführt und die Aufnahme mit und ohne Brillenglas jeweils miteinander verglichen.

In einer übergeordneten Anwendung können die verschiedenen vorstehend beschriebenen Verfahren, d.h. das erfindungsgemäße Verfahren sowie das wenigstens eine weitere Verfahren, kombiniert werden, um beispielsweise aus einem Vergleich der jeweils erhaltenen Ergebnisse eine höhere Genauigkeit oder eine Plausibiltätsüberprüfung der in den einzelnen Verfahren erhaltenen Ergebnisse zu erhalten. Die verschiedenen vorstehend beschriebenen Verfahren können nacheinander oder gleichzeitig in der übergeordneten Anwendung erfolgen. Erfolgen die verschiedenen Verfahren nacheinander kann deren Reihenfolge unabhängig voneinander sein und/oder es kann sich um eine beliebige Reihenfolge handeln. Erfolgen die verschiedenen Verfahren nacheinander kann es bevorzugt sein, wenigstens eines der vorstehend beschriebenen Verfahren zur Bestimmung der Brechkraftverteilung zuletzt durchzuführen. Eine übergeordnete Anwendung kann beispielsweise ein die verschiedenen Verfahren umfassendes Computerprogramm sein.

Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigen:

Figur 1 ein bevorzugtes Ausführungsbeispiel einer Vorrichtung zur Bestimmung eines Refraktionsfehlers eines Auges eines Nutzers; und

Figur 2 ein Ablaufdiagramm des erfindungsgemäßen Verfahrens zur Bestimmung des Refraktionsfehlers des Auges des Nutzers.

Ausführungsbeispiele

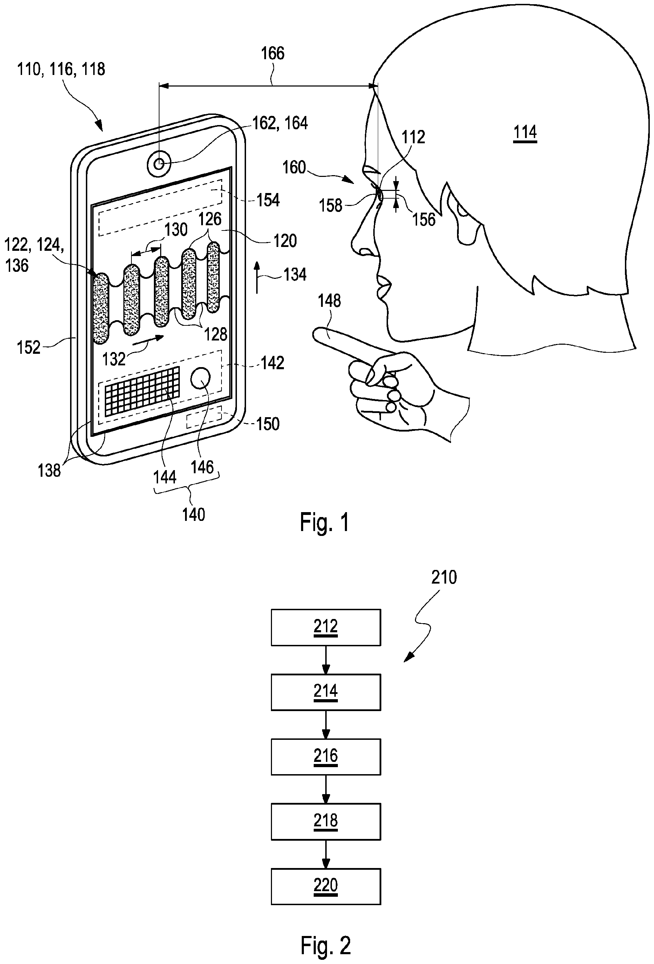

Figur 1 zeigt schematisch ein bevorzugtes Ausführungsbeispiel einer Vorrichtung 110 zur Bestimmung eines Refraktionsfehlers eines Auges 112 eines Nutzers 114. Die vorgeschlagene Vorrichtung 110 ist in der Darstellung gemäß Figur 1 und in der nachfolgenden Beschreibung - ohne Beschränkung der Allgemeinheit - als mobiles Kommunikationsgerät 116 in Form eines Smartphones 118 ausgeführt. Eine Ausführung der Vorrichtung 110 in Form eines anderen mobilen Kommunikationsgeräts 116, insbesondere als Mobiltelefon (Handy) oder Tablet, ist jedoch ebenfalls denkbar.

Die Vorrichtung 110 umfasst einen Bildschirm 120, der, wie aus Figur 1 hervorgeht, im Wesentlichen die Form eines Rechtecks annimmt. Der Bildschirm 120 ist zur Darstellung eines Zeichens 122 eingerichtet. Erfindungsgemäß stellt das Zeichen 122 ein Muster 124 dar, welches über eine graphische Struktur verfügt, die - insbesondere im Gegensatz zu einem Rauschen, das ohne erkennbare Struktur bleibt - über mindestens eine räumliche Periode verfügt, innerhalb der die Struktur des Musters 124 wiederholt dargestellt ist. Das Muster wird daher auch als periodisches Muster bezeichnet.

Der Bildschirm 120 ist weiterhin zur Darstellung einer Veränderung eines Parameters des auf dem Bildschirm dargestellten Zeichens 122 eingerichtet. Aufgrund der elektronischen Ansteuerung des Bildschirms 120 auf dem Smartphone 118 kann der ausgewählte Parameter des auf dem Bildschirm dargestellten Musters 124 auf einfache Weise und in einem weiten Rahmen verändert werden. Bei dem hier vorliegenden periodischen Muster 124 kann der Parameter bevorzugt auf eine Eigenschaft einer periodischen Funktion verknüpft sein. Hier kann insbesondere eine Wiederholfrequenz verwendet werden, mit welcher die Struktur derart wiederholt dargestellt werden kann, dass sich durch die Wiederholung gleichartige Punkte oder Bereiche über die Struktur des Musters 124 ausbilden können. In der Darstellung gemäß Figur 1 sind periodische Maxima 126 und Minima 128 als bevorzugte Ausgestaltungen gleichartiger Punkte oder Bereiche des Musters 124 erkennbar. Bei der hier verwendeten periodischen Funktion handelt es sich um eine Sinusfunktion. Andere periodische Funktionen, z.B. eine Cosinusfunktion oder einer Überlagerung einer Cosinusfunktion mit einer Sinusfunktion sind jedoch denkbar.

Erfindungsgemäß umfasst der Parameter des auf dem Bildschirm 120 dargestellten Zeichens mindestens eine Ortsfrequenz des periodischen Musters 124, wobei der Begriff der Ortsfrequenz einen Kehrwert eines räumlichen Abstands 130 zwischen benachbart angeordneten gleichartigen Punkten, insbesondere zwischen benachbarten Maxima 126 oder zwischen benachbarten Minima 128, in einer räumlich periodischen Änderung des Musters. Hierbei kann die Ortsfrequenz in der Einheit 1/m oder alternativ als dimensionslose Zahl "Einheiten pro Grad" oder "Zyklen pro Grad" angegeben werden. Wie in Figur 1 schematisch dargestellt, kann hierbei die Intensität des Musters 124 entlang einer ersten Richtung 132 der Ausdehnung des Bildschirms 120 dem Verlauf der periodischen Funktion, insbesondere der Sinusfunktion, folgen. Andere Arten der Bestimmung der Ortsfrequenz aus dem Muster sind jedoch denkbar, beispielsweise aus einem Abstand von Punkten gleicher Intensität.

Wie Figur 1 weiterhin zeigt, umfasst das periodische Muster in dieser besonders bevorzugten Ausführung eine zweidimensionale Überlagerung der periodischen Funktion, insbesondere der Sinusfunktion, welche sich in die erste Richtung 132 auf der Ausdehnung des Bildschirms 120 erstreckt, und einer konstanten Funktion, welche sich in eine zweite Richtung 134 auf der Ausdehnung des Bildschirms 120 erstreckt, die hier senkrecht zu der ersten Richtung 132 angeordnet ist. Andere Winkel zwischen der ersten Richtung 132 und der zweiten Richtung 134 sind jedoch ebenfalls möglich. Auf diese Weise kann das Muster 124 auf dem Bildschirm 120 in Form von periodisch nebeneinander angeordneten Streifen 136 vorliegen, die auch als "sinusförmiges Gitter" bezeichnet werden. Andere Arten von Mustern 124 sind jedoch ebenfalls möglich. Der Nutzer 114 betrachtet auf dem Bildschirm somit das sinusförmige Gitter, das die periodisch nebeneinander angeordneten Streifen 136 mit hohem Kontrast und einer Vielzahl an Ortsfrequenzen umfasst, in einer festgelegten Entfernung. Für die Entfernung kann vorzugsweise ein Wert von 25 cm bis 1 m, besonders bevorzugt von 40 cm bis 75 cm, insbesondere von 50 cm, gewählt werden.

Im Falle einer Kurzsichtigkeit (Myopie) des Nutzers 114 ist das Auge 112 defokussiert und für die Entfernung kann ein derartiger Wert eingestellt werden. Das gleiche erfolgt bei jungen myopen Nutzern, die eine Brille tragen. Bei einer Brille die eine vorliegende Fehlsichtigkeit ausreichend gut korrigiert, ist die Scheinauflösung sehr hoch. Ist die Brille jedoch nur teilweise korrigierend, ist das Auge 112 des Nutzers 114 defokussiert und für die Entfernung kann ein derartiger Wert eingestellt werden. Bei einem jungen weitsichtiger (hyperoper) Nutzer 114, kann auf diese Weise keine Messung erfolgen, da eine hohe Restakkommodation des Auges 112 des jungen Nutzers 114 keinen Nachweis der Defokussierung zulässt. In diesem Falle kann das Muster 124 in einer Entfernung von mindestens 4 m dargestellt werden; das Smartphone 118 kann hierbei als Eingabeeinheit verwendet werden.

Wie weiterhin aus Figur 1 erkennbar ist, kann das Muster 124 derart auf der Ausdehnung des Bildschirms 120 dargestellt werden, dass die erste Richtung 132 und die die zweite Richtung 134 jeweils parallel zu einer Orientierung des Bildschirms 120 parallel zu einem Rand 138 des Bildschirms 120, der in der Darstellung gemäß Figur 1 im Wesentlichen die Form eines Rechtecks annimmt, angeordnet sein können. Auf diese Weise kann das Muster 124 an die vorhandene Ausdehnung des Bildschirms 120 angepasst sein. Andere Arten der Darstellung des Musters 124 auf dem Bildschirm 120 sind jedoch denkbar, beispielsweise unter einem Winkel von 45° ± 15° oder einem ungeraden Vielfachen davon in Bezug auf die Orientierung des Bildschirms 120.

Wie weiterhin in Figur 1 dargestellt ist, kann die ausgewählte periodische Funktion mit einer weiteren Funktion überlagert werden. In der Darstellung gemäß Figur 1 ist die Sinusfunktion in Bezug auf die erste Richtung 132 derart mit einer abnehmenden Funktion überlagert, dass die Ortsfrequenz des periodischen Musters 124 entlang der ersten Richtung 132 abnimmt. Auf diese Weise kann die Darstellung des Musters 124 auf dem Bildschirm 120 bereits eine Reihe von Ortsfrequenzen aufweisen, die hier in abfallender Reihe entlang der ersten Richtung 132 angeordnet sind.

Die Vorrichtung 110 umfasst weiterhin eine Eingabeeinheit 140, die zur Erfassung einer Reaktion des Nutzers 114 in Abhängigkeit von dem auf dem Bildschirm 120 dargestellten Zeichen 122 eingerichtet ist. Insbesondere kann die Erfassung der Reaktion des Nutzers 114 während monokular erfolgen, bevorzugt nacheinander für jedes der beiden Augen 112 des Nutzers 114, wobei der Nutzer 114 jeweils das andere, nicht verwendete Auge 112 abdecken kann. Hierbei kann bevorzugt zuerst das rechte Auge 112 und anschließend das linke Auge 112 des Nutzers 114 zur Erfassung seiner Reaktion verwendet werden. Der Nutzer kann hierbei zu einem Wechsel des Auges 112 zur Betrachtung des Zeichens 122 auf dem Bildschirm 120 bevorzugt durch eine entsprechende Menüführung auf dem Smartphone 118 veranlasst werden.

Um dem Nutzer 114 zu ermöglichen, eine gewünschte Antwort auf einen Stimulus des Auges 112 des Nutzers 114 in Folge der Darstellung des Zeichens 122 auf dem Bildschirm 120 zu ermöglichen, kann das Smartphone 118 in der Ausführung gemäß Figur 1 einen Eingabebereich 142 aufweisen, welcher auf dem berührungsempfindlichen Bildschirm 120 (Touchscreen) des Smartphones 118 dargestellt ist. Der Eingabebereich 142 kann hierbei die Form einer virtuellen Tastatur 144 annehmen. Alternativ oder zusätzlich kann der Eingabebereich 142 jedoch eine andere Art der Ausgestaltung, zum Beispiel in Form eines Buttons 146, aufweisen. Weiterhin alternativ kann jedoch auch vorgesehen sein, dass die Eingabeeinheit 140 außerhalb des Bildschirms 120 der Vorrichtung 110 angebracht ist. Alternativ oder zusätzlich kann die Eingabeeinheit 140 weiterhin zur Aufnahme einer Spracheingabe eingerichtet sein, mittels welcher der Nutzer 114 die Eingabe seiner Reaktion an die Vorrichtung 110 übermitteln kann.

Unabhängig von der tatsächlichen Art der Ausführung der Eingabeeinheit 140 kann der Nutzer 114 somit, vorzugsweise durch eine manuelle Beaufschlagung der Eingabeeinheit 140, insbesondere mittels eines Fingers 148 des Nutzers 114, die Eingabeeinheit 140 derart bedienen, dass die Eingabeeinheit 140 in Folge der Beaufschlagung der Eingabeeinheit 140 durch den Nutzer 114 ein Messsignal erzeugt, das an eine Auswerteeinheit 150 der Vorrichtung 110 weitergeleitet werden kann. In einer bevorzugten Ausführung kann der Nutzer 114 nun diejenige Ortsfrequenz einstellen, bei der er gerade noch einen schwarzweiß Kontrast erkennen kann; dies entspricht einem ersten Nulldurchgang der auf dem Bildschirm 120 dargestellten Sinusfunktion. Dies kann insbesondere dadurch erfolgen, dass zu Beginn eine hohe Ortsfrequenz dargestellt wird und diese dann schrittweise verringert wird, oder in dem zu Beginn eine geringe Ortsfrequenz dargestellt wird, die dann schrittweise erhöht wird. Hierbei kann die Einstellung des Wertes für die Ortsfrequenz unabhängig vom Nutzer 114 vorgegeben werden. Alternativ oder zusätzlich kann dem Nutzer 114, insbesondere mittels Betätigung der Eingabeeinheit 140, die Möglichkeit gegeben werden, selbst Einfluss auf die Ortsfrequenz, die auf dem Bildschirm 120 dargestellt ist, zu nehmen. Darüber hinaus kann eine Information, ob der Nutzer 114 den Bildschirm 120 mit oder ohne Sehhilfe betrachtet, vorzugsweise ebenfalls durch Betätigung der Eingabeeinheit 140, mit erfasst werden.

Wie in Figur 1 schematisch dargestellt, kann die Vorrichtung 110 weiterhin ein Gehäuse 152 aufweisen, welches die Auswerteeinheit 150 umfassen kann. Alternativ oder zusätzlich kann die Auswerteeinheit 150 jedoch auch außerhalb des Gehäuses 152 angebracht sein, wobei eine drahtgebundene oder eine drahtlose Verbindung (nicht dargestellt) zwischen der Eingabeeinheit 140 und der Auswerteeinheit 150 vorgesehen sein kann. Weitere Arten der Ausführung sind jedoch möglich.

Erfindungsgemäß ist die Auswerteeinheit 150 dazu eingerichtet, einen Zeitpunkt festzustellen, zu dem sich aus der Reaktion des Nutzers 114 eine Erkennbarkeit des auf dem Bildschirm 120 dargestellten Zeichens 122 für den Nutzer 114 ergibt, worunter zu verstehen ist, dass der Nutzer 114 die auf dem Bildschirm dargestellte Ortsfrequenz des periodischen Musters 124 gerade noch oder gerade erst erkennen kann. Hierzu kann die Ortsfrequenz in dem periodischen Muster 124 zeitlich und/oder räumlich, insbesondere entlang der ersten Richtung 132, zunehmen oder abnehmen.

Gleichzeitig wird der Nutzer 114 dazu angehalten, durch eine Bedienung der Eingabeeinheit 140 anzugeben, dass er die auf dem Bildschirm dargestellte Ortsfrequenz des periodischen Musters 124 gerade noch oder gerade erst erkennen kann. Um die Reaktion des Nutzers 114 möglichst auf die gewünschte Weise zu erhalten, kann ein Darstellungsteil 154 des Bildschirms 120 oder, alternativ oder zusätzlich, eine akustische Ausgabeeinheit (nicht dargestellt) dazu verwendet werden, um den Nutzer 114 entsprechend zu informieren oder zu der gewünschten Reaktion anzuhalten.

Erfindungsgemäß ist die Auswerteeinheit 150 weiterhin dazu eingerichtet, einen Wert für den Refraktionsfehler des Auge 112 des Nutzers 114 aus einer Angabe des Zeitpunkts zu bestimmen, zu dem sich aus der Reaktion des Nutzers 114 ergibt, dass der Nutzer 114 die auf dem Bildschirm dargestellte Ortsfrequenz des periodischen Musters 124 gerade noch oder gerade erst erkennen kann. Hierzu wird das Messsignal, das der Nutzer 114 während Schritt b) durch das Bedienen der Eingabeeinheit 140 erzeugt, an die Auswerteeinheit 150 weitergeleitet, die dazu eingerichtet ist, hieraus den gewünschten Zeitpunkt festzustellen. Weiterhin ist aufgrund der der elektronischen Ansteuerung des Bildschirms 120 auf dem Smartphone 118 die Ortsfrequenz des auf dem Bildschirm 120 dargestellten periodischen Musters 124 bekannt und kann somit von der Auswerteeinheit 150 zur gewünschten Auswertung verwendet werden. In einer besonders bevorzugten Ausführung kann die Auswerteeinheit 150 hierzu weiterhin dazu eingerichtet sein, den gewünschten Parameter des Zeichen 122, insbesondere die Ortsfrequenz des periodischen Musters 124, durch Ansteuerung des Bildschirms 120 einzustellen.

Zur Bestimmung des Wertes für den Refraktionsfehler des Auge 112 des Nutzers 114 aus der zu dem Zeitpunkt festgelegten Ortsfrequenz des periodischen Musters 124 wird erfindungsgemäß auf die oben dargestellte Bestimmung der Scheinauflösung des Auges 112 des Nutzers 114 zurückgegriffen. In der Ausführung der vorliegenden Erfindung gemäß Figur 1 kann hierzu wie oben beschrieben vorgegangen werden. Gemäß der oben angegebenen Gleichung (2)

$$Defokus\,[D] = \frac{21{,}3}{Ortsfrequenz \cdot Pupillendurchmesser\,[m]} \qquad (2)$$

kann die Defokussierung in Dioptrien D ermittelt werden, die in erster Näherung dem sphärischen Äquivalent der gesuchten Korrektion entspricht.

Gemäß Gleichung (2) ist die Defokussierung jedoch von einem Pupillendurchmesser 156 einer Pupille 158 im Auge 112 des Nutzers 114 abhängig. Als geschätzter Wert für den Pupillendurchmesser 156 kann ein durchschnittlicher Durchmesser der Pupille 156 bei Tag von 2 bis 3 mm verwendet werden. Vorzugsweise kann der Pupillendurchmesser 156 jedoch messtechnisch erfasst werden. Hierzu kann, insbesondere während der Nutzer 114 das sinusförmige Gitter auf dem Bildschirm 120 des Smartphones 118 betrachtet, ein Bild einer Augenpartie 160 des Nutzers 114 aufgenommen werden. Wie in Figur 1 schematisch dargestellt, kann hierzu bevorzugt eine Kamera 162 verwendet werden, wobei es sich bei der Kamera 162 vorzugsweise um eine Frontkamera 164 des Smartphones 118 handeln kann.

Mittels der Kamera 162 kann somit das gewünschte Bild der Augenpartie 160 des Nutzers 114 an jedem beliebigen Ort aufgenommen werden. Insbesondere mittels Bildverarbeitung, die vorzugsweise durch die Auswerteeinheit 150 durchgeführt werden kann, können aus dem aufgenommenen Bild geometrische Daten der Pupille 158, insbesondere eine Lage und der Durchmesser 156 der Pupille 158 im Auge 112 des Nutzers 114, ermittelt werden.

Bei bekannter Ortsfrequenz des auf dem Bildschirm 120 dargestellten periodischen Musters 124 und bei bekanntem Pupillendurchmesser 156 kann somit unter Verwendung von Gleichung (2) die Defokussierung des Auges 112 des Nutzers 114 in Dioptrien D ermittelt werden, die, wie oben angegeben, in erster Näherung dem sphärischen Äquivalent der gesuchten Korrektion entspricht. In einer weiteren Ausführung kann zusätzlich ein als Pupillendistanz 166 bezeichneter Abstand zwischen der Kamera 162 und dem Auge 112 des Nutzers 114 ermittelt werden. Zur Bestimmung der Pupillendistanz 166 kann eine Abstandsmessung durchgeführt werden, bevorzugt eine Abstandsmessung, über die das Smartphone 118 bereits verfügt. Alternativ oder zusätzlich kann die Pupillendistanz 166 durch Triangulation über eine bekannte Pixelanzahl der Kamera 162 bei Detektion eines bekannten Gegenstandes oder Bildinhaltes durch die Kamera 162 bestimmt werden.

Wie bereits erwähnt, kann durch die Ermittlung der Scheinauflösung in erster Näherung das sphärische Äquivalent der Korrektion bestimmt werden. Die Scheinauflösung kann jedoch auch in mindestens zwei Meridianen bestimmt werden, vorzugsweise indem das periodische Muster 124 gemäß Figur 1 auf dem Bildschirm 120 zunächst entlang der ersten Richtung 132 und hieran anschließend (nicht dargestellt) entlang der zweiten Richtung 134, die vorzugsweise senkrecht zu der ersten Richtung 132 angeordnet ist auf dem Bildschirm 120 dargestellt wird. Auf diese Weise können für das sphärozylindrische Brillenglas mit astigmatischer Wirkung nacheinander die Scheitelbrechwerte jedes der beiden Hauptschnitte, die senkrecht zueinander stehen, ermittelt werden. Für weitere Details hierzu wird auf WO 2018/077690 A1 verwiesen.

Figur 2 zeigt schematisch ein Ablaufdiagramm eines bevorzugten Ausführungsbeispiels eines erfindungsgemäßen Verfahrens 210 zur Bestimmung des Refraktionsfehlers des Auges 112 des Nutzers 114.

In einem Darstellungsschritt 212 erfolgt hierzu gemäß Schritt a) die Darstellung des periodischen Muster 124 auf dem Bildschirm 120, wobei die eine Ortsfrequenz des auf dem Bildschirm 120 dargestellten periodischen Musters 124 verändert wird.

In einem Erfassungsschritt 214 erfolgt gemäß Schritt b) die Erfassung der Reaktion des Nutzers 114 in Abhängigkeit von der gemäß dem Darstellungsschritt 212 auf dem Bildschirm 120 dargestellten Ortsfrequenz des periodischen Musters 124.

In einem Feststellungsschritt 216 erfolgt gemäß Schritt c) die Feststellung des Zeitpunkts, zu dem sich aus der Reaktion des Nutzers 114 in dem Erfassungsschritt 214 eine Erkennbarkeit des auf dem Bildschirm 120 dargestellten Zeichens 122 für den Nutzer 114 ergibt, so dass der Nutzer 114 die gemäß dem Darstellungsschritt 212 auf dem Bildschirm 120 dargestellte Ortsfrequenz des periodischen Musters 124 gerade noch oder gerade erst erkennen kann.

In einem Bestimmungsschritt 218 erfolgt gemäß Schritt d) die Bestimmung eines Wertes 220 für den Refraktionsfehler des Auges 112 des Nutzers 114 aus der Ortsfrequenz des periodischen Musters 124, welche zu dem Zeitpunkt, der in dem Feststellungsschritt 216 ermittelt wurde, zur Darstellung des periodischen Musters 124 auf dem Bildschirm 120 in dem Darstellungsschritt 212 festgelegt war.

Bezugszeichenliste

| | |
|---|---|
| 110 | Vorrichtung |
| 112 | Auge |
| 114 | Nutzer |
| 116 | mobiles Kommunikationsgerät |
| 118 | Smartphone |
| 120 | Bildschirm |
| 122 | Zeichen |
| 124 | Muster |
| 126 | Maximum |
| 128 | Minimum |
| 130 | räumlicher Abstand |
| 132 | erste Richtung |
| 134 | zweite Richtung |
| 136 | Streifen |
| 138 | Rand |
| 140 | Eingabeeinheit |
| 142 | Eingabebereich |
| 144 | Tastatur |
| 146 | Button |
| 148 | Finger |
| 150 | Auswerteeinheit |
| 152 | Gehäuse |
| 154 | Darstellungsteil |
| 156 | Pupillendurchmesser |
| 158 | Pupille |
| 160 | Augenpartie |
| 162 | Kamera |
| 164 | Frontkamera |
| 166 | Pupillendistanz |
| 210 | Verfahren zur Bestimmung eines Refraktionsfehlers eines Auges eines Nutzers |
| 212 | Darstellungsschritt |
| 214 | Erfassungsschritt |
| 216 | Feststellungsschritt |
| 218 | Bestimmungsschritt |
| 220 | Wert eines Refraktionsfehlers eines Auges eines Nutzers |

**Patentansprüche**

**1.** Verfahren (210) zur Bestimmung eines Refraktionsfehlers eines Auges (112) eines Nutzers (114), wobei das Verfahren (210) die folgenden Schritte umfasst:

a) Darstellen eines Zeichens (122) auf einem Bildschirm (120), wobei ein Parameter des auf dem Bildschirm (120) dargestellten Zeichens (122) verändert wird;

b) Erfassen einer Reaktion des Nutzers (114) in Abhängigkeit von dem auf dem Bildschirm (120) dargestellten Zeichen (122);

c) Feststellen eines Zeitpunkts, zu dem sich aus der Reaktion des Nutzers (114) eine Erkennbarkeit des auf dem Bildschirm (120) dargestellten Zeichens (122) für den Nutzer (114) ergibt; und

d) Bestimmen eines Wertes (220) für den Refraktionsfehler des Auges (112) des Nutzers (114) aus dem zu dem Zeitpunkt festgelegten Parameter,

**dadurch gekennzeichnet,**
**dass** das auf dem Bildschirm (120) dargestellte Zeichen (122) ein periodisches Muster (124) ist, wobei der Parameter des auf dem Bildschirm (120) dargestellten Musters (124) mindestens eine Ortsfrequenz umfasst, und dass der Wert (220) für den Refraktionsfehler aus der zu dem Zeitpunkt festgelegten Ortsfrequenz des Musters (124) bestimmt wird.

2. Verfahren (210) nach dem vorangehenden Anspruch, **dadurch gekennzeichnet, dass** die Ortsfrequenz des Musters (124) vergrößert oder verkleinert wird.

3. Verfahren (210) nach einem der vorangehenden Ansprüche, **dadurch gekennzeichnet, dass** das Muster (124) aus einer Überlagerung einer periodischen Funktion und einer konstanten Funktion gebildet wird.

4. Verfahren (210) nach dem vorangehenden Anspruch, **dadurch gekennzeichnet, dass** die periodische Funktion ausgewählt ist aus einer Sinusfunktion, einer Cosinusfunktion oder einer Überlagerung hiervon.

5. Verfahren (210) nach einem der beiden vorangehenden Ansprüche, **dadurch gekennzeichnet, dass** die periodische Funktion zusätzlich derart mit einer zunehmenden Funktion oder einer abnehmenden Funktion überlagert wird, dass die Ortsfrequenz des Musters (124) in einer Richtung zunimmt oder abnimmt.

6. Verfahren (210) nach dem vorangehenden Anspruch, **dadurch gekennzeichnet, dass** die Richtung einen Winkel in Bezug auf eine Orientierung des Bildschirms (120) einnimmt, wobei der Winkel 0° oder ein Vielfaches von 90° ist.

7. Verfahren (210) nach einem der vorangehenden Ansprüche, **dadurch gekennzeichnet, dass** das Muster zunächst in einer ersten Richtung (132) dargestellt wird und anschließend in einer gegen die erste Richtung veränderten zweiten Richtung (134) dargestellt wird.

8. Verfahren (210) nach dem vorangehenden Anspruch, **dadurch gekennzeichnet, dass** die jeweilige Ortsfrequenz des Musters (124) in die erste Richtung (132) und in die zweite Richtung (134) zu einer Bestimmung einer sphärozylindrischen Korrektion verwendet wird.

9. Verfahren (210) nach einem der vorangehenden Ansprüche, **dadurch gekennzeichnet, dass** der Wert (220) des Refraktionsfehlers des Auges (112) des Nutzers (114) einer Defokussierung des Auges (112) des Nutzers (114) entspricht, wobei die Defokussierung als *Defokus* [D] in Dioptrien *D* gemäß Gleichung (2)

$$Defokus\,[D] \; = \; \frac{21{,}3}{Ortsfrequenz \cdot Pupillendurchmesser\,[m]} \quad (2)$$

bestimmt wird, wobei die Ortsfrequenz, welche der Nutzer (114) gerade noch oder gerade erst erkennen kann, als dimensionslose Zahl angegeben wird, und wobei das Auge (112) des Nutzers (114) den Pupillendurchmesser (156) in m aufweist.

10. Verfahren (210) nach dem vorangehenden Anspruch, **dadurch gekennzeichnet, dass** der Pupillendurchmesser (156) des Auges (112) des Nutzers (114) messtechnisch erfasst wird.

11. Verfahren (210) nach dem vorangehenden Anspruch, **dadurch gekennzeichnet, dass** der Pupillendurchmesser (156) des Auges (112) des Nutzers (114) durch Aufnahme eines Bildes des Auges (112) des Nutzers (114) mittels einer Kamera (162), durch Bildverarbeitung des Bildes und durch Bestimmung einer Pupillendistanz (166) zwischen der Kamera (162) und dem Auge (112) des Nutzers (114) ermittelt wird.

12. Computerprogramm zur Bestimmung eines Refraktionsfehlers eines Auges (112) eines Nutzers (114), wobei das Computerprogramm dazu eingerichtet ist, die folgenden Verfahrensschritte durchzuführen:

a) Darstellen eines Zeichens (122) auf einem Bildschirm (120), wobei ein Parameter des auf dem Bildschirm (120) dargestellten Zeichens (122) verändert wird;
b) Erfassen einer Reaktion des Nutzers (114) in Abhängigkeit von dem auf dem Bildschirm (120) dargestellten Zeichen (122);
c) Feststellen eines Zeitpunkts, zu dem sich aus der Reaktion des Nutzers (114) eine Erkennbarkeit des auf dem Bildschirm (120) dargestellten Zeichens (122) für den Nutzer (114) ergibt; und
d) Bestimmen eines Wertes (220) für den Refraktionsfehler des Auges (112) des Nutzers (114) aus dem zu dem Zeitpunkt festgelegten Parameter,

**dadurch gekennzeichnet,**
**dass** das Computerprogramm ferner dazu eingerichtet ist, um auf dem Bildschirm (120) ein periodisches Muster (124) als Zeichen (122) darzustellen, wobei der Parameter des auf dem Bildschirm (120) dargestellten Zeichens (122) mindestens eine Ortsfrequenz des Musters (124) umfasst, und den Wert (220) für den Refraktionsfehler des Auges (112) des Nutzers (114) aus der zu dem Zeitpunkt festgelegten Ortsfrequenz des Musters (124) zu bestimmen.

13. Verfahren zur Herstellung eines Brillenglases für ein Auge (112) eines Nutzers (114), wobei die Herstellung des Brillenglases durch Bearbeitung eines Brillenglasrohlings erfolgt, wobei der Brillenglasrohling anhand von Zentrierdaten bearbeitet wird, wobei die Zentrierdaten Anweisungen zum Ausgleich eines Refraktionsfehlers des Auges (112) des Nutzers (114) umfassen, wobei eine Bestimmung des Refraktionsfehlers des Auges (112) des Nutzers (114) die folgenden Schritte umfasst:

a) Darstellen eines Zeichens (122) auf einem Bildschirm (120), wobei ein Parameter des auf dem Bildschirm (120) dargestellten Zeichens (122) verändert wird;
b) Erfassen einer Reaktion des Nutzers (114) in Abhängigkeit von dem auf dem Bildschirm (120) dargestellten Zeichen (122);
c) Feststellen eines Zeitpunkts, zu dem sich aus der Reaktion des Nutzers (114) eine Erkennbarkeit des auf dem Bildschirm (120) dargestellten Zeichens (122) für den Nutzer (114) ergibt; und
d) Bestimmen eines Wertes (220) für den Refraktionsfehler des Auges (112) des Nutzers (114) aus dem zu dem Zeitpunkt festgelegten Parameter,

**dadurch gekennzeichnet,**
**dass** das auf dem Bildschirm (120) dargestellte Zeichen (120) ein periodisches Muster (124) ist, wobei der Parameter des auf dem Bildschirm dargestellten Zeichens (122) mindestens eine Ortsfrequenz des Musters (124) umfasst, und dass der Wert (220) für den Refraktionsfehler des Auges (112) des Nutzers (114) aus der zu dem Zeitpunkt festgelegten Ortsfrequenz des Musters (124) bestimmt wird.

14. Vorrichtung (110) zur Bestimmung eines Refraktionsfehlers eines Auges (112) eines Nutzers (114), wobei die Vorrichtung umfasst:

- einen Bildschirm (120), der zur Darstellung eines Zeichen (122) und einer Veränderung eines Parameters des Zeichens (122) eingerichtet ist;
- eine Eingabeeinheit (140), die zur Erfassung einer Reaktion des Nutzers (114) in Abhängigkeit von dem auf dem Bildschirm (120) dargestellten Zeichen (122) eingerichtet ist; und
- eine Auswerteeinheit (150), die zur Feststellung eines Zeitpunkts, zu dem sich aus der Reaktion des Nutzers (114) eine Erkennbarkeit des auf dem Bildschirm (120) dargestellten Zeichens (122) für den Nutzer (114) ergibt, und zur Bestimmung eines Wertes (220) für den Refraktionsfehler des Auges (112) des Nutzers (114) aus dem zu dem Zeitpunkt festgelegten Parameter eingerichtet ist,

**dadurch gekennzeichnet,**
**dass** Bildschirm (120) zur Darstellung eines periodischen Musters (124) als das Zeichen (122) eingerichtet ist, wobei der Parameter des auf dem Bildschirm (120) dargestellten Zeichens (122) mindestens eine Ortsfrequenz des Musters (124) umfasst, und dass die Auswerteeinheit (150) zur Bestimmung des Wertes (220) für den Refraktionsfehler des Auges (112) des Nutzers (114) aus der zu dem Zeitpunkt festgelegten Ortsfrequenz des Musters (124) eingerichtet ist.

15. Vorrichtung nach dem vorangehenden Anspruch, **dadurch gekennzeichnet, dass** die Vorrichtung weiterhin eine Kamera (162) umfasst, wobei die Kamera (162) dazu eingerichtet ist, ein Bildes des Auges (112) des Nutzers (114) aufzunehmen, und wobei die Auswerteeinheit (150) weiterhin dazu eingerichtet ist, durch Bildverarbeitung des Bildes des Auges (112) des Nutzers (114) und durch Bestimmung einer Pupillendistanz (166) zwischen der Kamera (162) und dem Auge (112) des Nutzers (114) eine Ermittlung des Pupillendurchmessers (156) des Auges (112) des Nutzers (114) auszuführen.

Fig. 1

Fig. 2

EP 3 730 036 A1

**Europäisches Patentamt**
**European Patent Office**
**Office européen des brevets**

# EUROPÄISCHER RECHERCHENBERICHT

Nummer der Anmeldung

EP 19 17 0558

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/019779 A1 (LEGERTON JEROME A [US] ET AL) 26. Januar 2012 (2012-01-26) * Absätze [0021] - [0029], [0037] - [0040] * ----- | 1-15 | INV. A61B3/103 A61B3/032 |
| X | US 2013/176534 A1 (FRANKFORT BENJAMIN J [US] ET AL) 11. Juli 2013 (2013-07-11) * Absätze [0039] - [0066] * ----- | 1-15 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| München | 22. Oktober 2019 | Martelli, Luca |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

20

**EP 3 730 036 A1**

## ANHANG ZUM EUROPÄISCHEN RECHERCHENBERICHT
## ÜBER DIE EUROPÄISCHE PATENTANMELDUNG NR.

EP 19 17 0558

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-10-2019

| Im Recherchenbericht angeführtes Patentdokument | Datum der Veröffentlichung | Mitglied(er) der Patentfamilie | Datum der Veröffentlichung |
|---|---|---|---|
| US 2012019779 A1 | 26-01-2012 | US 7918558 B1<br>US 2012019779 A1<br>WO 2012012757 A1 | 05-04-2011<br>26-01-2012<br>26-01-2012 |
| US 2013176534 A1 | 11-07-2013 | US 2013176534 A1<br>US 2015150443 A1 | 11-07-2013<br>04-06-2015 |

EPO FORM P0461

Für nähere Einzelheiten zu diesem Anhang : siehe Amtsblatt des Europäischen Patentamts, Nr.12/82

21

**EP 3 730 036 A1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2014268060 A1

- WO 2018077690 A1

**In der Beschreibung aufgeführte Nicht-Patentliteratur**

- **NACH L. N. ; THIBOS, W. WHEELER ; D. HORNER.** Power Vectors: An Application of Fourier Analysis to the Description and Statistical Analysis of Refractive Error. *Optometry and Vision Science,* 1997, vol. 74 (6), 367-375

- **M. YOUNG.** *Optik, Laser, Wellenleiter,* 1997
- **NACH F. SCHAEFFEL ; A. DE QUEIROZ.** Alternative Mechanisms of Enhanced Underwater Vision in the Gartner Snakes Tamnophis melanogaster and T. couchii. *Copeia,* 1990, vol. 1, 50-58